# EUROPEAN PATENT SPECIFICATION

(11) **EP 1 480 927 B1**
(45) Date of publication and mention of the grant of the patent: **26.12.2012**
(21) Application number: 03706599.2
(22) Date of filing: 04.03.2003
(51) Int. Cl.: C07B 61/00

(54) **A METHOD FOR CLEAVAGE OF LABILE FUNCTIONAL GROUPS FROM CHEMICAL COMPOUNDS**
VERFAHREN ZUR SPALTUNG VON LABILEN FUNKTIONELLEN GRUPPEN VON CHEMISCHEN VERBINDUNGEN
PROCEDE DE CLIVAGE DE GROUPES FONCTIONNELS LABILES A PARTIR DE COMPOSES CHIMIQUES

(30) Priority: 04.03.2002 DE 10209203; 04.03.2002 US 361562 P
(43) Date of publication of application: 01.12.2004
(73) Proprietor: Universität Konstanz, 78457 Konstanz (DE); Steiner, Ulrich, 78465 Konstanz (DE); Wöll, Dominik, 78464 Konstanz (DE)
(72) Inventor: STEINER, Ulrich, 78465 Konstanz (DE); WÖLL, Dominik, 78464 Konstanz (DE); WALBERT, Stefan, 78464 Konstanz (DE)
(74) Representative: Bösl, Raphael Konrad
(86) International application number: PCT/EP2003/002208
(87) International publication number: WO 2003/074542

(56) References cited:
- WO-A-01/32671
- WO-A2-2004/001033
- LEPROUST E ET AL: "DIGITAL LIGHT-DIRECTED SYNTHESIS A MICROARRAY PLATFORM THAT PERMITS RAPID REACTION OPTIMIZATION ON ACOMBINATORIAL BASIS" JOURNAL OF COMBINATORIAL CHEMISTRY, AMERICAN CHEMICAL SOCIETY, WASHINGTON, US, vol. 2, no. 4, 11 July 2000 (2000-07-11), pages 349-354, XP001058977 ISSN: 1520-4766
- QIAN, XUHONG ET AL: "Interaction of naphthyl heterocycles with DNA: effects of thiono and thio groups" PERKIN 2 (2000), (4), 715-718 , XP002252650
- SLOCUM, GREGORY H. ET AL: "Photochemistry of naphthylmethyl halides. Direct and sensitized paths to homolytic and heterolytic carbon-halogen bond cleavage" JOURNAL OF ORGANIC CHEMISTRY (1984), 49(12), 2177-85 , XP002252651
- PIRRUNG M C ET AL: "PROOFING OF PHOTOLITHOGRAPHIC DNA SYNTHESIS WITH 3',5'-DIMETHOXYBENZOINYLOXYCARBONYL-PROTEC TED DEOXYNUCLEOSIDE PHOSPHORAMIDITES" JOURNAL OF ORGANIC CHEMISTRY, AMERICAN CHEMICAL SOCIETY. EASTON, US, vol. 63, 1998, pages 241-246, XP000916248 ISSN: 0022-3263 cited in the application
- SANGEET SINGH-GASSON ET AL: "Maskless fabrication of light-directed oligonucleotide microarrays using a digital micromirror array" NATURE BIOTECHNOLOGY, NATURE PUBLISHING, US, vol. 17, October 1999 (1999-10), pages 974-978, XP002126301 ISSN: 1087-0156 cited in the application

## Description

The present invention relates to a method for cleavage of labile functional groups from molecules by the action of electromagnetic radiation as well as to a method for preparing DNA chips by spatially addressed, light-controlled nucleotide synthesis on solid substrates, further to a chemical composition as well as to the use of said chemical composition in preparing DNA chips.

Easy cleavage of functional groups from molecules plays an important role in many fields of chemistry and biology, for example, in the construction of larger chemical units, such as in the synthesis of polymers, natural products, etc. In doing so, particularly reactive groups which may affect or disturb the respective intended linking of two molecules by undesired side reactions, are temporarily "masked" or protected in a selective manner by chemically or physically cleavable functional protective groups to prevent them from participating in the desired linking reaction.

The use of large combinatorial libraries of binding partners, immobilized on a substrate, of the biomolecules provided in solution is of great advantage in a comparative study of molecular recognition between biomolecules of the same or different structural classes.

To the person skilled in the art, the term "biomolecules" designates compounds of the classes of nucleic acids and their derivatives, proteins, peptides and carbohydrates.

This principle of mutual molecular recognition is applied, in particular, in the selective construction of polynucleotides from nucleoside units and/or oligonucleotide units. The selective construction of polynucleotides, in turn, is of decisive importance in preparing DNA chips having a high density ("high-density DNA-chip") of polynucleotides arranged thereon.

DNA chips, i.e. so-called microarrays of fields on a glass or polymer substrate of immobilized DNA or randomly selected oligonucleotides functioning as super-multiplex probes for molecular recognition by hybridization, (S.P.A. Fodor, Science 277 (1997) 393, DNA Sequencing Massively Parallel Genomics) have long since been employed, for example, in medicine and in pharmaceutical research.

In said fields, DNA chips again play an important role in genetic analysis and diagnostics. The most wide-spread technique for preparing such DNA chips is the so-called spatially addressed, parallel, light-controlled oligonucleotide synthesis on solid substrates (see e.g. S.P.A. Fodor et al, Nature 364 (1993) 555, Multiplexed Biochemical Arrays with Biological chips) using photolabile protective groups, i.e. protective groups for reactive functionalities of nucleoside or nucleotide units which can be selectively cleaved again under the action of, in most cases, UV light of a certain wavelength, so that the protected functionalities are available again for further reaction. Thymidin derivatives with photolabile groups are known from WO0132671.

In this case, the afore-mentioned, so-called photolithographic technique serves to prepare said DNA chips. For this purpose, the synthetic construction of the desired oligonucleotide chains on the substrate is controlled by suitable labile protective groups, which release the linkage site for the respective next nucleotide, for example, upon exposure to light. These protective groups have hitherto been preferably photolabile. With the help of these photolabile protective groups, a combinatorial strategy can be developed by means of spatially selective exposure to light, said strategy allowing to generate extremely dense, spatially addressable microarrays of oligonucleotides whose number increases exponentially with the number of synthesis cycles ("split and pool"). For a currently achievable surface area of each element of less than 50 (µm)², more than 10⁶ probe fields may be theoretically arranged on 1 (cm)². Light exposure has hitherto been effected with the help of micromirror arrays (S. Singh-Gasson, et al, Nature Biotechn. 17 (1999) 974, Maskless Fabrication of Light Directed Olignucleotide Microarrays using a Digital Micromirror Array), as used in digital projection technology. This saves the time- and cost-intensive preparation of exposure masks and has made it possible to prepare the DNA chips more rapidly using said photolithographic technique.

Currently employed photolabile protective groups do not yield satisfactory results in terms of the error rate of the thus-synthesized DNA chips (D.J. Lockheart and E.A. Winseler, Nature 405 (2000) 827, Genomics, Gene expression and DNA arrays). Cleavage of the protective groups does not take place sufficiently completely; moreover, there are disturbing side reactions with undesired reaction products, so that a large number of the oligonucleotides on the DNA chips can not be used.

A central point in photolithographic synthesis consists in the use of the photolabile protective groups, which can be used in numerous chemical variants in organic and bioorganic chemistry (V.N.R. Pillay, Photolithic Deprotection and Activation of Functional Groups; in: Organic Photochemistry, Vol. 9 ed. A. Padwa (Marcel Dekker, New York and Basel, 1987), page 225 et seq.). The most common protective groups are photolabile protective groups on the basis of the 2-nitrobenzyl group. (J.E.T. Corrie and D.R. Trentham, Caged Nucleotides and Neurotransmitters; in: Biological Applications of Photochemical Switches, in: Bioorganic Photochemistry Series, Vol. 2 ed. Harry Morrison (Wiley Interscience, 1993), page 243 et seq.).

In the preparation of DNA chips, for example when protecting the terminal 5'-OH group in the oligonucleotide construction from the 3' end to the 5' end or from the 5' end to the 3' end, the preferred protective group among the protective groups of the 2-nitrobenzyl type has hitherto been, above all, the MeNPOC (α-methyl-nitropiperonyloxycarbonyl) protective group which has long since been the standard protective group in DNA chip preparation (S.P.A. Fodor, et al, Science 251 (1991), 767, Light Directed, Spatially Adressable Parallel Chemical Synthesis).

A disadvantage of this type of protective groups is the formation, upon irradiation, of the aromatic nitrosoketone which represents a very reactive leaving group. This results in undesired consequent reactions which often cause errors in the nucleotide structure of the resulting oligonucleotide or polynucleotide.

Use has recently been made also of the DMBOC group (3',5'-dimethoxy benzoinyloxycarbonyl) as a protective group (M.C.Pirrung et al, J.Org. Chem. 63 (1998), 241, Proofing of Photolithographic DNA synthesis with 3',5'-Dimethoxybenzoinyloxycarbonyl-protected Deoxynucleosidephosphoramidites) in selective polynucleotide synthesis. The cleavage of the DMBOC protective group produces a benzofurane derivative whose intense fluorescence may be used, for example, as an indicator of the progress of the photoreaction.

Both of the presently known photolabile protective groups require irradiation times of several minutes under the usual irradiation intensities with the 365 nm line of a mercury lamp, in order to react quantitatively.

Further, 2-(2-nitrophenyl)-ethoxycarbonyl compounds are known in the preparation of DNA chips, wherein the protective groups are cleaved as 2-nitrostyrene derivatives (German Patent Nos. 44 44 996 and 196 20 170, and US-5,763,599). Also, these compounds are somewhat less prone to error than the aforementioned compounds in terms of disturbing side reactions due to cleavage of generally somewhat less reactive 2-nitrostyrenes.

Therefore, it was an object of the present invention to find a method wherein the cleavage of labile groups was considerably reduced in terms of reaction time of the cleavage reaction and allowing the cleavage reaction to be optimised in terms of its yield. A further object was to reduce the risk of undesired side reactions during cleavage of the labile protective group.

The above object of the present invention is achieved in that the method, according to the invention, for cleavage of labile functional groups from molecules comprises the following steps:
a) taking Thioxanthone
b) contacting it with the molecules comprising said labile functional groups,
   wherein said molecules are molecules of following formula: 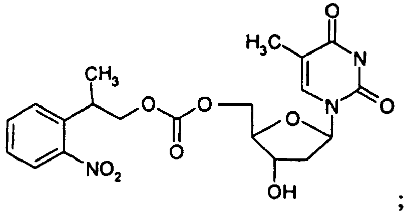
c) exposure to electromagnetic radiation, the sequence of steps b) and c) being optional, wherein in case step c) is carried out prior to step b), the chemical compound Thioxanthone is activated by said electromagnetic radiation.

The method according to the invention allows to achieve excitation of the triplet state of the selected chemical compound Thioxanthone and to transfer the electromagnetic radiation thus absorbed by the selected chemical compound (also termed as "sensitizer") to the labile functional group via a triplet-triplet transition, which labile functional group may be subsequently cleaved in an efficient and rapid manner.

This finding is particularly surprising, especially in the synthesis of nucleic acids, which are instable when brought together with the radicals which are generated in the method according to the invention during the excitation of the sensitizer. The sensitizer in its excited state is a radical. It is well known in the art that many polymers, especially biopolymers are instable when reacting with or brought in contact with radicals. Especially nucleic acids will react with radicals via a radical substitution at the position of the bases or by breaking nucleic acids at its sugar positions in small fragments. The cleavage of a nucleic acid yields to free nucleic bases (adenine, cytosine, guanine and thymine) which are the reaction product of the radicalic substitution of the bases of nucleic acid. The scission of nucleic acids leads to a large variety of different nucleic acid fragments thus not yielding the desired biopolymer. (See e.g. G.H. McGall et al., "New Insight into the Mechanism of Base Propenal Formation during Bleomycin-Mediated DNA Degradation", J. Am. Chem. Soc. 1992, 114, 4958-4967 and P. E. Nielsen et al., "DNA Binding and Photocleavage by Uranyl(VI) (UO22+) Salts", J. Am. Chem Soc. 1992,114,4967-4975).

Similar reactions are known from other biopolymers like e.g. oligopeptides, proteins and sugars (carbohydrates).

Therefore, the method according to the invention provides a surprising finding that those polymers, being destroyed during or after formation can be synthesized according to the method of the invention by using radicalic sensitizers, which overcomes a long prejudice in the art.

The term "very similar triplet state" as used herein means that "very similar" comprises an energy range around the triplet state which is a multiple n of the average thermal energy RT with an order of magnitude of ca. 2.5 kJ of the triplet state of the chemical compound according to the invention, wherein n=8, preferably n=4.

The sequence of steps b) - c) makes particularly good use of the radiation energy, since, in addition to the conventional reaction of the photolabile group, sensitizing via triplet-triplet energy transfer is also used.

The contacting is effected by methods essentially known to the person skilled in the art. Both compounds, i.e. the molecules comprising the labile functional groups and the chemical compound Thioxanthone (also referred to in the following as "sensitizer or sensitizer compound"), are advantageously present in the same phase.

However, cleavage is also successful where only the sensitizer compound is activated by said electromagnetic radiation, i.e. for a sequence of steps c) - b) of the method according to the invention. The sensitizer compound then transfers its triplet energy very efficiently to the functional protective group. This advantageously results in molecules being subjected to a cleavage reaction which would otherwise be unsuitable for such reactions, which are provided with functional protective groups and which would be destroyed, for example, by electromagnetic radiation having a defined wavelength initiating cleavage. This is effected by taking thioxanthone and irradiating it at an uncritical wavelength which does not destroy the molecules and only activates the sensitizer compound which subsequently transfers said energy, by means of said triplet-triplet transfer, to the sensitive molecules, so that the functional groups can be cleaved nevertheless. For example, the sensitizer compound can be activated by prior radiation with a laser or other high-energy radiation, such as X-ray, electron or particle beams, such as a or γ-rays.

Also, a further advantageous embodiment allows steps b) and c) to be carried out simultaneously.

The labile group is photolabile, so that the method according to the invention can be easily employed, in particular, in known methods for the preparation of DNA chips. The electromagnetic radiation is preferably within the wavelength range of UV/VIS radiation (210 - 450 nm). This allows the method according to the invention to be used preferably in the preparation of DNA chips using conventional mercury vapour lamps.

It is disclosed that, the singlet state of the chemical compound thioxanthone is as high as or lower than the singlet state of the labile functional group. Under these prerequisites, the wavelength, and thus the energy of the incident light may be shifted within a certain range, i.e. to a so-called "window" of the electromagnetic spectrum, in which the side reactions to be expected, in particular in preparing the DNA chips, can be minimized.

It is disclosed that, the triplet-singlet energy gap of the chemical compound Thioxanthone is smaller than the triplet-singlet energy gap of the labile functional group.

The object of the above invention is further achieved by a method for preparing molecular libraries containing biomolecules, in particular for preparing DNA chips and peptide chips as well as their analogous and mimetic forms, by means of spatially addressed light-controlled synthesis on solid substrates, said method comprising the following steps:
a) reacting the unprotected terminal 5' hydroxy group of a nucleoside located on the solid substrate under usual conditions with a photolabile protective group and optionally purifying the reaction product,
   wherein the reaction product is a molecule of following formula: 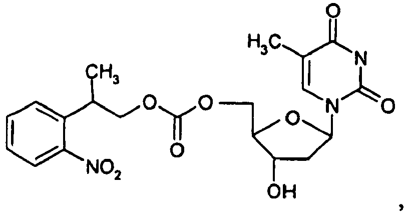
b) applying Thioxanthone on the surface of the carrier, said surface comprising the nucleosides modified in step a);
c) irradiating, in a spatially selective manner, the surface of the carrier treated in step b), with electromagnetic radiation in the UV/VIS range;
d) reacting with the molecule produced in step a);
e) optionally repeating steps b) to d).

The compound Thioxanthone is applied by common methods, such as knife coating, atomising, spraying, dropwise addition, etc., wherein the chemical compound may be added in a pure state, in solution, as a suspension or as a dispersion.

This results in an increase in the overall absorption of electromagnetic radiation, since the light absorbed by the sensitizer has a very effective impact on the cleavage reaction due to the transfer of its triplet energy to the labile protective group. At a given radiation intensity, cleavage, therefore, takes place faster in the presence of the sensitizer.

Further, the object of the present invention is achieved by providing a chemical composition which comprises a molecule of formula: 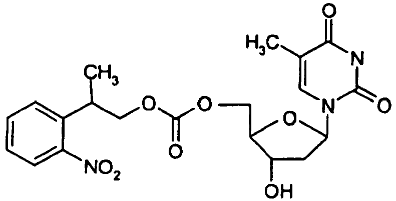 and Thioxanthone.

The combination of the two different compounds allows a virtually loss-free transfer of triplet excitation energy from Thioxanthone to the labile functional group, which picks up said energy and is then more easily cleaved without itself having to be excited by electromagnetic radiation.

The abbreviations have the following meanings herein:

| | |
|---|---|
| NPPOC | 2-(2-nitrophenyl)propyloxycarbonyl |
| MeNPPOC | 2-(3, 4-methylenedioxy-2-nitrophenyl) propyloxycarbonyl |
| MeNPOC | 2-(3, 4-methylenedioxy-2-nitrophenyl) oxycarbonyl |
| DMBOC | Dimethoxybenzoinylyloxycarbonyl |
| NPES | 2-(2-nitrophenyl)ethylsulfonyl |
| NPPS | 2-(2-nitrophenyl)propylsulfonyl |
| PhNPPOC | 2-(5-phenyl-2-nitrophenyl)-propyloxycarbonyl |

The nucleoside bases guanine, thymine, cytosine and adenosine are represented by their usual abbreviations G, T, C and A.

Use is made of thioxanthen-9-one (thioxenthone), as chemical Compound.

Use is preferably made of solutions comprising, based on the solvent employed, 0.001 to 5 weight-%, particularly preferably between 0.005 and 0.05 weight-% of the chemical compound Thioxanthone.

In many cases, an amount of more than 5 weight-% of the chemical compound (sensitizer) Thioxanthone leads to unwanted chemical reactions. One reaction is the reaction between an excited sensitizer and a non-excited sensitizer molecule. The sensitizer is a radical in its excited state.

The other reaction often encountered is the reaction of the excited sensitizer Thioxanthane with the molecule comprising the photolabile group described above and may destroy them. Therefore, lower concentrations are preferred in most cases. The precise selection of the suitable concentration is easy to determine by the person skilled in the art using few prior experiments known per se to the person skilled in the art.

The chemical composition according to the invention is preferably used for preparing DNA chips, since it allows an easy transfer of energy between the triplet state of the sensitizer compound and of the photolabile protective group and thus the photochemical cleavage reaction can be initiated in a particularly rapid and complete manner.

According to the invention, the term "nucleotide" means both polynucleotides comprising two to 10 nucleosides which are linked with each other via both 3'-5' and 5'-3' phosphoric acid ester bonds. However, the nucleotides according to the invention also comprise polynucleotides consisting of more than 10 nucleoside units.

The methods according to the invention are suitable for DNA- nucleotide synthesis.

The methods according to the invention are particularly suitable to carry out an automated method. Such automated method is preferably embodied as a parallel synthesis for preparing a nucleotide library.

In a further embodiment, the present invention comprises a kit comprising the chemical composition of the present invention containing some or all reagents and/or auxiliaries and/or solvents and/or a working instruction for carrying out a method according to the invention in one spatial unit, said kit comprising at least one or more selected nucleotides.

According to a still further embodiment, the present invention comprises the use of the method according to the invention preparing of oligonucleotides or nucleic acid chips, preferably for the automated manufacture of oligonucleotides or nucleic acid chips.

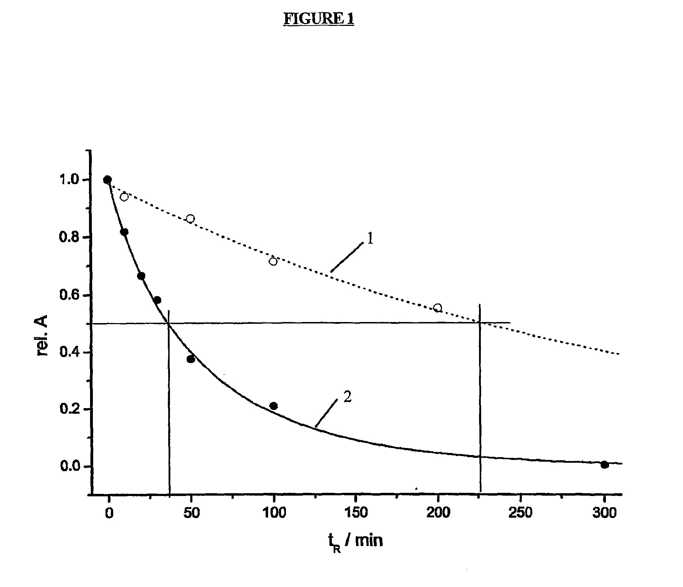

Figure 1 shows the reaction rate of the cleavage reaction in a method according to the invention.

In Fig. 1 the abscissa indicates the relative concentration of the compound provided with a protective group, as determined by HPLC, plotted as a function of the radiation time (t_{R}/min) in minutes (ordinate). Graph 1 represents the radiation of compound T07 (2-(5-iodo-2-nitrophenyl)propylthymidine-5'-yl-carbonate) (0.096 mM) at 366 nm and an intensity of 4.98 x 10⁻³ W/cm⁻². Graph 2 shows the radiation of compound T02 (2-(2-chloro-6-nitrophenyl)ethylthymidine-5'-yl-carbonate) (0.091 mM) at the same wavelength and an intensity of 6.39 x 10⁻³ W/cm⁻² in the presence of thioxanthone (0.113 mM) as the sensitizer in ammonia-saturated acetonitrile. Although the light intensity in the case of Experiment 2 is only 25% higher, the reaction rate is six times as high.

**Table 1: Molecule containing a protolabile protective group used in a method according to the invention**

| |
|---|
| 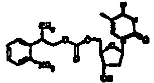 |
| T0₂ |

The triplet state of Thioxantone is above the triplet state of the compound comprising the photolabile protective group, and is at most energetically similar to the latter. Thus, the singlet-triplet energy gap of the sensitizer is preferably smaller than that of the labile protective group.

The chemical compound Thioxanthone further has a high triplet formation quantum yield φ_{T}, which linearly enters as a factor in sensitizing efficiency.

**Table 3:**

| | | | | | |
|---|---|---|---|---|---|
| **Chemical compounds Thioxanthone ("sensitizer compound") used in a method according to the invention :** | | | | | |

| Compound | E(S₁) kJ/mol | εₘₐₓ M⁻¹xcm⁻¹ | E(T₁) kJ/mol | φ_{T} | τ_{T} |
|---|---|---|---|---|---|
| | | | | | |
| Thioxanthone | p | 6800 | 265 | <0.88 | 73 µs |
| 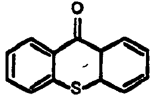 | n/b | (380 nm) | | | 95 µs |

The energy (E) of the singlet and triplet states is indicated in kJ/mol. The absorption coeffcient ε is indicated as M⁻¹ x cm⁻¹ at the respective wavelength. n refers to a non-polar solvent, p refers to a polar solvent, b is a benzene-like solvent.

τ refers to the lifetime of the triplet state in µs.

### Experimental conditions:

The term "conventional conditions known to the person skilled in the art " as used hereinbefore and hereinafter, is described, for example in US-5,763,599 or in DE 4,444,956.

UV-VIS-absorption measurements were carried out using a UV-VIS spectrometer Lambda 18 (Perkin-Elmer) running under the UV Winlab software, fluorescence measurements were carried out using a luminescence spectrometer LS 50 (Perkin-Elmer) running under the FL Winlab software.

The radiation equipment consisted of a high-pressure mercury lamp (200 W), a heat filter (optical path length: 5 cm, filled with 0.3 M CuSO₄ solution in water), a collecting lens, an electronically controlled shutter, a 366 nm interference filter (Schott) and a temperature-controlled cell holder for light exposure cells (Hellma QS, 1 cm).

HPLC examinations were carried out using Merck-Hitachi equipment. Said equipment consisted of an L-7100 pump, an L-7200 autosampler, an L-7450A UV-diode array detector and an L-7000 interface. Merck LiChrospher 100 RP-18 (5 µm) was used as the column. Control was effected using the HSM manager on a Compaq computer.

### Examples

### Example 1:

### Reaction for cleavage of a labile functional group from a molecule in solution by means of the method according to the invention

3 ml of a solution of thymidine derivative T02 (Tablet 1) (0.091 mM) and thioxanthone (0.113 mM) was pipetted into a cuvette and flushed for ca. 15 minutes with ammonia by passing the gas through the solution. Said solution was irradiated with light at a wavelength of 366 nm for different periods of time. Absorption spectra were respectively measured before and after radiation.

The solution was then flushed with nitrogen (saturated with acetonitrile) for ca. 15 minutes. Upon said nitrogen flushing, an absorption spectrum was measured again, and the solution was finally separated into its components in HPLC. These components were characterized using a UV-diode array detector.

### Example 2

### Preparation of DNA chips (not claimed)

The deprotection reaction was carried out under standard test conditions for DNA chip syntheses in a MAS 2.0 or 3.0 of Nimblegen Systems, Madison, USA. The design of the DNA chips to be prepared exhibited a standard array as usually employed in quality control tests.

There was a typical density of some 10,000 to 100,000 oligomers per (cm)², mostly present as 18-25 mers. The size or the surface area of an individual synthesis spot was 35 µm x 35 µm. Said spot consisted of the image (1:1) of 4 micromirrors arranged in a square (Texas Instrument Digital Light Processor) (each mirror having an edge length of 16 µm x 16 µm), each mirror being spaced apart by 1 µm.

0.01 weight-% thioxanthone was used as sensitizer, based on the solvent employed, i.e. DMSO. This resulted in a reduction of the light dose until complete cleavage of the protective group, from 7.5 W/cm², without a sensitizer molecule, to a value of 3 W/cm² at an effective lamp power of ca. 0.2 - 0.6 W/cm². The lamp power is independent of the MAS-type and is determined during radiation in each case.

| | | | |
|---|---|---|---|
| MAS-type | 2.0: | 1,000 Watts | Hg lamp |
| MAS-type | 3.0 | 200 Watts | Hg lamp |

### Duration of the deprotection reaction:

| | Lamp power W/cm² | Duration (s) |
|---|---|---|
| Without thioxanthone | 0.2 | 37.5 |
| Without thioxanthone | 0.6 | 12.5 |
| With thioxanthone (0.01 wight-%) | 0.2 | 15 |
| With thioxanthone (0.01 weight-%) | 0.6 | 5 |

## Claims

1. A method for cleavage of photolabile functional groups from molecules by the action of electromagnetic radiation which method comprises the following steps:
a) taking Thioxanthone;
b) contacting it with the molecules comprising said photolabile functional groups, wherein said molecules are molecules of following formula: 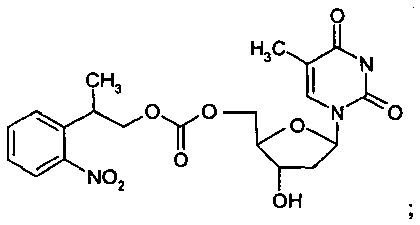
c) exposure to electromagnetic radiation, the sequence of steps b) and c) being optional, wherein in case step c) is carried out prior to step b), the chemical compound Thioxanthone is activated by said electromagnetic radiation.

2. The method as claimed in Claim 1 **characterized in that** step c) and step b) are carried out at the same time.

3. The method as claimed in any one of Claims 1 and 2, **characterized in that** the electromagnetic radiation is radiation in the wavelength region of UV/VIS radiation.

4. Use of the method as claimed in any one of the preceding Claims for preparing spatially addressed molecule libraries.

5. A method for preparing molecular libraries containing biomolecules, in particular for preparing DNA chips, by means of spatially addressed, light-controlled synthesis from individual structural units of the biomolecules on solid substrates, which method comprises the following steps:
a) reacting the unprotected terminal 5' hydroxy group of a nucleoside located on the solid substrate under usual conditions with a photolabile protective group, and optionally purifying the reaction product, wherein the reaction product is a molecule of following formula: 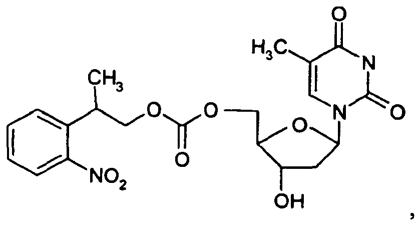
b) applying Thioxanthone, on the surface of the carrier comprising the nucleosides modified in step a),
c) irradiating, in a spatially selective manner, the surface of the carrier treated in step b), with electromagnetic radiation in the UVNIS range;
d) reacting with the molecule produced in step a);
e) optionally repeating steps b) to d).

6. A chemical composition, comprising a molecule of formula: 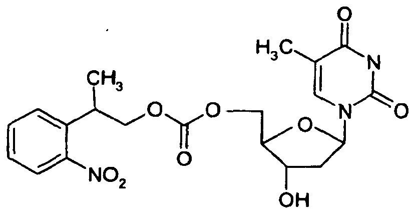 and Thioxanthone.

7. The use of the chemical composition of Claim 6 for the manufacture of DNA chips.

8. A kit comprising the chemical composition of Claim 6.

9. A kit according to claim 8 containing, in one spatial unit, part of or all reagents and/or auxiliaries and/or solvents and/or a working instruction which are for carrying out a method as claimed in any one of Claims 1 to 3 and/or 5.

10. Use of the method as claimed in claim 5 for preparing oligonucleotides, or nucleic acid chips.

11. Use of the method as claimed in any one of Claims 1 to 3 for preparing spatially addressed molecular libraries.

12. Use of a kit as claimed in Claim 8 and/or 9 in a method of any of claims 1 to 3 for preparing spatially addressed molecular libraries.

## Patentansprüche

1. Verfahren zum Abspalten von photolabilen funktionellen Gruppen von Molekülen durch die Einwirkung von elektromagnetischer Strahlung, wobei das Verfahren die folgenden Schritte umfasst:
a) Nehmen von Tioxanthon;
b) Inkontaktbringen davon mit den Molekülen, die die photolabilen funktionellen Gruppen umfassen, wobei die Moleküle Moleküle der folgenden Formel sind: 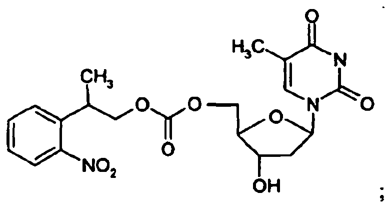
c) elektromagnetische Bestrahlung, wobei die Abfolge der Schritte b) und c) optional ist, wobei in dem Fall, dass Schritt c) vor Schritt b) ausgeführt wird, die chemische Verbindung Thioxanthon durch die elektromagnetische Strahlung aktiviert wird.

2. Verfahren, wie in Anspruch 1 beansprucht, **dadurch gekennzeichnet, dass** der Schritt c) und der Schritt b) zur selben Zeit ausgeführt werden.

3. Verfahren, wie in einem der Ansprüche 1 und 2 beansprucht, **dadurch gekennzeichnet, dass** die elektromagnetische Strahlung Strahlung im Wellenlängenbereich von UV/VIS-Strahlung ist.

4. Verwendung des Verfahrens, wie in einem der vorhergehenden Ansprüche beansprucht, zur Herstellung von räumlich adressierten ("spatially addressed") Molekülbibliotheken.

5. Verfahren zur Herstellung von molekularen Bibliotheken, enthaltend Biomoleküle, insbesondere zur Herstellung von DNA-Chips, mittels räumlich adressierten, lichtkontrollierter Synthese der Biomoleküle auf festen Substraten von individuellen Struktureinheiten, wobei das Verfahren die folgenden Schritte umfasst:
a) Umsetzen der nicht geschützten terminalen 5'-Hydroxygruppe eines Nukleosids, das auf dem festen Substrat vorhanden ist, unter gewöhnlichen Bedingungen mit einer photolabilen Schutzgruppe und gegebenenfalls Reinigung des Reaktionsprodukts, wobei das Reaktionsprodukt ein Molekül der folgenden Formel ist: 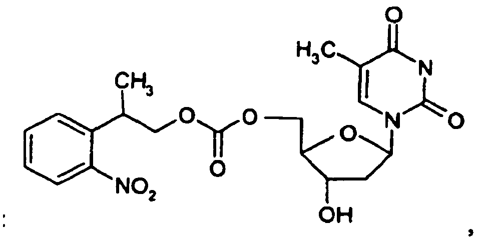
b) Auftragen von Thioxanthon auf die Oberfläche des Trägers, der die in Schritt a) modifizierten Nukleoside umfasst,
c) Bestrahlung der Oberfläche des in Schritt b) behandelten Trägers mit elektromagnetischer Strahlung im UV/VIS-Bereich in einer räumlich selektiven Weise;
d) Umsetzen des in Schritt a) produzierten Moleküls;
e) gegebenenfalls Wiederholen der Schritte b) bis d).

6. Chemische Zusammensetzung, umfassend ein Molekül der Formel: 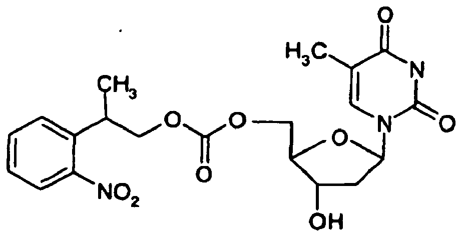 und Thioxanthon.

7. Die Verwendung der chemischen Zusammensetzung von Anspruch 6 zur Herstellung von DNA-Chips.

8. Kit, umfassend die chemische Zusammensetzung von Anspruch 6.

9. Kit gemäß Anspruch 8, enthaltend in einer räumlichen Einheit einen Teil oder alle Reagenzien und/oder Hilfsstoffe und/oder Lösungsmittel und/oder eine Arbeitsanleitung, die zum Ausführen eines Verfahrens sind, wie in einem der Ansprüche 1 bis 3 und/oder 5 beansprucht.

10. Verwendung des Verfahrens, wie in Anspruch 5 beansprucht, zur Herstellung von Oligonukleotiden oder Nukleinsäure-Chips.

11. Verwendung des Verfahrens, wie in einem der Ansprüche 1 bis 3 beansprucht, zur Herstellung von räumlich adressierten molekularen Bibliotheken.

12. Verwendung eines Kits, wie in Anspruch 8 und/oder 9 beansprucht, in einem Verfahren nach einem der Ansprüche 1 bis 3 zur Herstellung von räumlich adressierten molekularen Bibliotheken.

## Revendications

1. Procédé de clivage de groupes fonctionnels photolabiles de molécules par l'action d'un rayonnement électromagnétique, lequel procédé comprend les étapes suivantes :
a) prise de Thioxanthone ;
b) mise en contact avec les molécules comprenant lesdits groupes fonctionnels photolabiles, dans laquelle lesdites molécules sont des molécules de la formule suivante : 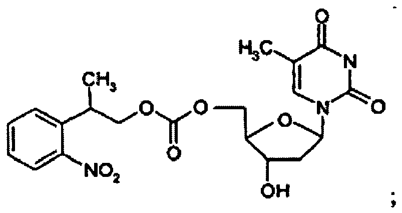
c) l'exposition au rayonnement électromagnétique, la séquence des étapes b) et c) étant facultative, où dans le cas où l'étape c) est conduite avant l'étape b), le composé chimique Thioxanthone est activé par ledit rayonnement électromagnétique.

2. Procédé tel que revendiqué selon la revendication 1, **caractérisé en ce que** l'étape c) et l'étape b) sont conduites au même moment.

3. Procédé tel que revendiqué selon l'une quelconque des revendications 1 et 2, **caractérisé en ce que** le rayonnement électromagnétique est un rayonnement de la région des longueurs d'onde du rayonnement UV/VIS.

4. Utilisation du procédé tel que revendiqué selon l'une quelconque des revendications précédentes, pour la préparation de bibliothèques de molécules spatialement traitées.

5. Procédé de préparation de bibliothèques moléculaires contenant des molécules biologiques, en particulier pour la préparation de puces à ADN, à l'aide de la synthèse traitée spatialement, contrôlée par la lumière à partir de motifs structurels individuels de molécules biologiques sur des substrats solides, lequel procédé comprend les étapes suivantes :
a) réaction du groupe hydroxy non protégé en position terminale 5' d'un nucléoside situé sur le substrat solide sous des conditions habituelles avec un groupe protecteur photolabile, et éventuellement la purification du produit réactionnel, dans laquelle le produit réactionnel est une molécule de la formule
suivante : 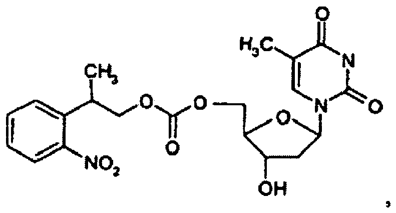
b) application de Thioxanthone, sur la surface du support comprenant les nucléosides modifiés dans l'étape a),
c) irradiation, d'une manière spatialement sélective, de la surface du support traité dans l'étape b), avec un rayonnement électromagnétique dans la plage des UV/VIS ;
d) réaction avec la molécule produite dans l'étape a) ;
e) éventuellement la répétition des étapes b) à d).

6. Composition chimique, comprenant une molécule de formule : 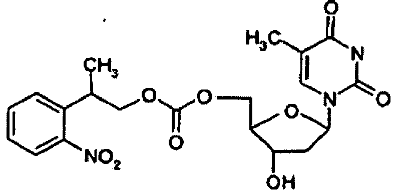 et de Thioxanthone.

7. Utilisation de la composition chimique selon la revendication 6 pour la fabrication de puces à ADN.

8. Trousse comprenant la composition chimique selon la revendication 6.

9. Trousse selon la revendication 8 contenant, dans un motif spatial, une partie ou tous les réactifs et/ou les auxiliaires et/ou les solvants et/ou une instruction de travail qui servent à conduire un procédé tel que revendiqué selon l'une quelconque des revendications 1 à 3 et/ou 5.

10. Utilisation du procédé tel que revendiqué selon la revendication 5 pour la préparation d'oligonucléotides, ou de puces à acides nucléiques.

11. Utilisation du procédé tel que revendiqué selon l'une quelconque des revendications 1 à 3 pour la préparation de bibliothèques moléculaires spatialement traitées.

12. Utilisation d'une trousse telle que revendiquée selon la revendication 8 et/ou 9 dans un procédé selon l'une quelconque des revendications 1 à 3 pour la préparation de bibliothèques moléculaires spatialement traitées.
